# EUROPEAN PATENT APPLICATION

(11) **EP 1 611 889 A1**
(43) Date of publication of application: **04.01.2006**
(21) Application number: 04719056.6
(22) Date of filing: 10.03.2004
(51) Int. Cl.: A61K 31/407, A61K 31/403, A61P 31/04, A61P 35/00, C07D 487/08

(54) **ANTIBACTERIAL AGENT AND ANTICANCER AGENT**

(30) Priority: 10.03.2003 JP 2003063741; 10.10.2003 JP 2003352970
(71) Applicant: MITSUBISHI CORPORATION, Tokyo 100-8086 (JP); Mashino, Tadahiko, Minato-ku, Tokyo 105-8512 (JP)
(72) Inventor: MASHINO, Tadahiko, Kyoritsu University of Pharmacy, Minato-ku, Tokyo 105-8512 (JP)
(74) Representative: Calamita, Roberto
(86) International application number: PCT/JP2004/003082
(87) International publication number: WO 2004/080455

(57) **Abstract**

A novel antimicrobial agent and anticancer agent comprising, as an active ingredient, a fullerene derivative having a high antimicrobial activity and anticancer activity, wherein the antimicrobial agent or anticancer agent comprising, as an active ingredient, a fullerene derivative which has an organic bond structure represented by the following formula: 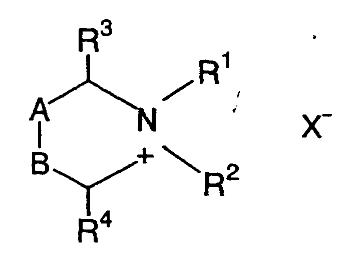
(wherein A and B denote adjacently bonded carbon atoms, R¹ and R² are identical or different and denote a hydrocarbon group which may have a substituent, R³ and R⁴ are identical or different and denote a hydrogen atom or a hydrocarbon group which may have a substituent, and X denotes an anion) attached to respective plural pairs of adjacent bonded carbon atom pairs constituting the carbon cluster skeleton of the fullerene, are provided.

## Description

### Technical Field

The invention of this application relates to an antimicrobial agent and anticancer agent. More particularly, the invention of this application relates to a novel antimicrobial agent and anticancer agent comprising a fullerene derivative as an active ingredient.

### Background Art

Fullerene, which is a carbon cluster represented by C60, is a novel carbon allotrope discovered by Smally, Kroto et al. in 1985. Since a large-scale synthesis method was established in 1990, studies have dramatically progressed in both basic and application fields.

At first, fullerenes were thought to have low chemical reactivity, however, it was shown that various anionic substrates are easily added thereto as an electron deficient olefin. In addition, Diels-Alder reaction, 1,3-dipolar cyclo addition reaction or the like proceeds well to give an adduct attached to the double bond of a conjunction region of 6-membered ring and 6-membered ring of a fullerene.

An effect of such a chemically active novel substance group on a living organism also attracts the interest. For example, the antimicrobial activity of a fullerene derivative of the following formula: 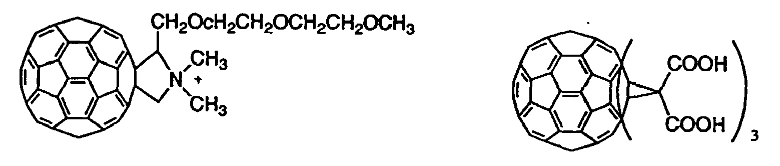
has been reported (non-patent literature 1), and also a fullerene derivative of the following formula: 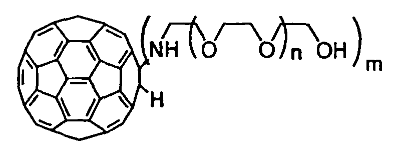
has been proposed to be an anticancer agent (patent literature 1).

However, with regard to the foregoing antimicrobial activity, the activity is not substantially too high, and the effect on antimicrobial-resistant bacteria against such as vancomycin is not clear. In addition, with regard to the foregoing anticancer agent, since fullerenes generate active oxygen under photo irradiation, by accumulating a fullerene derivative, to which the foregoing polymer is bonded, in a cancer cell, and by irradiating the cancer cell with light or ultrasound, active oxygen is locally generated to kill the cancer cell. In such a photo chemotherapy, a porphyrin derivative has been already applied to a drug and has been used, however, the indication is limited to a solid cancer to which light can be directly irradiated, namely skin cancer, esophageal cancer, stomach cancer or the like.

In this circumstance, a research on a fullerene derivative, which has a higher antimicrobial activity, or an anticancer fullerene derivative, which does not require photo irradiation and is applicable to a wide variety of cancers, has been desired, however, in fact, an investigation for application as a bioactive agent such as an antimicrobial agent or an anticancer agent as mentioned above has not so much progressed. As one of the reasons of this, there is a problem of the solubility.

Therefore, the inventor of this application synthesized two types of C60 fullerene derivatives represented by the following formulae (1) and (2): 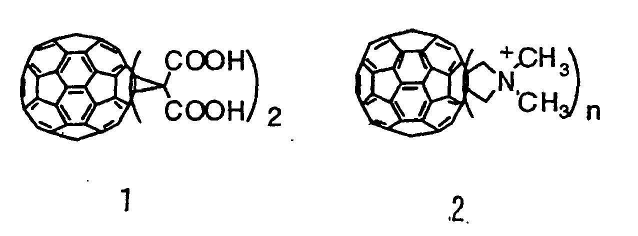
and has investigated their bioactivities. These derivatives have a characteristic that they easily dissolve in an organic solvent (such as DMSO), which is miscible in water, and can be used for a reaction in an aqueous solution.

Although human beings have been exposed to infectious diseases caused by various bacteria, by developing antimicrobial agents, a life span is extended, and the cause of death has changed to diseases derived from cancers or vascular damages. However, harmful bacteria to human beings have acquired resistance to currently used antimicrobial agents, which has become a social problem such as a hospital acquired infection. In such a background, it is considered that a novel antimicrobial agent having a new structure and mechanism which are different from those of a conventional antimicrobial agent can overcome the drug resistance of bacteria, therefore, the development has been desired. A fullerene derivative has a structure different from that of a conventional organic compound, and has a distinctive physical property derived from it, therefore, it is expected as a new drug against drug-resistant bacteria.

In addition, cancers have come to account for a large proportion of the current causes of death, and various anticancer agents have been developed. However, the problems of the current anticancer agents are that they have strong adverse effects and drug-resistant cancer cells emerge. With regard to a fullerene derivative having a characteristic as mentioned above, it is expected to provide a new anticancer agent against a wide variety of cancers which has a mild adverse effect and is less likely to cause drug resistance.
Patent literature 1: JP-A-9-235235
Non-patent literature 1: S. Bosi, et al., Bioorg. Med. Chem. Lett., 10,1043-1045 (2000), N. Taso, et al., J. Antimicro. Chemo., 49, 641-649 (2002)

Based on the background as above, the invention of this application makes it an object to provide a novel antimicrobial agent and anticancer agent comprising, as an active ingredient, a fullerene derivative having a high antimicrobial activity and anticancer activity.

### Disclosure of the invention

To solve the foregoing problems, the invention of this application provides firstly an antimicrobial agent or anticancer agent, characterized by comprising, as an active ingredient, a fullerene derivative which has an organic bond structure represented by the following formula: 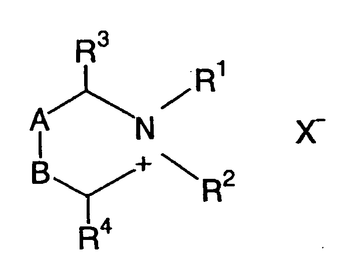
(wherein A and B denote adjacently bonded carbon atoms, R¹ and R² are identical or different and denote a hydrocarbon group which may have a substituent, R³ and R⁴ are identical or different and denote a hydrogen atom or a hydrocarbon group which may have a substituent, and X denotes an anion) attached to respective plural pairs of adjacent bonded carbon atom pairs constituting the carbon cluster skeleton of the fullerene.

In addition, with regard to the foregoing invention, the invention of this application provides secondly the antimicrobial agent or anticancer agent, characterized in that the hydrocarbon group is a linear chain, branched chain or cyclic hydrocarbon group, thirdly the antimicrobial agent or anticancer agent, characterized in that the hydrocarbon group is an alkyl group having 1 to 16 carbon atoms, fourthly the antimicrobial agent or anticancer agent, characterized in that the anion is at least one kind of inorganic acid ions and organic acid ions, and fifthly the antimicrobial agent or anticancer agent, characterized in that the fullerene is at least one kind of C60 fullerene and higher order fullerenes.

### Best Mode for Carrying Out the Invention

The invention of this application has characteristics as described above, and the embodiments will be explained below.

An active ingredient of the antimicrobial agent or anticancer agent of the invention of this application is a fullerene derivative having a structure represented by the foregoing formula. The fullerene derivative has a major characteristic that it has plural pairs of the foregoing organic bond structures relative to the pairs of adjacent bonded carbon atoms (A-B). Namely, the plural pairs are 2 pairs, 3 pairs, 4 pairs or the like.

In the foregoing formula representing the fullerene derivative of the invention of this application, the symbols R¹ and R² are identical or different and denote a hydrocarbon group which may have a substituent, R³ and R⁴ are identical or different and denote a hydrogen atom or a hydrocarbon group which may have a substituent. Here, as the hydrocarbon group, various kinds of linear or cyclic ones and various kinds of saturated or unsaturated ones are taken into account. Various kinds of aliphatic, alicyclic and aromatic hydrocarbons are taken into account.

Among them, as the hydrocarbon group, a linear chain or a branched chain aliphatic hydrocarbon group is exemplified. In this case, the carbon number is not limited, however, for example, one having about 1 to 16 carbon atoms is preferably exemplified.

The above hydrocarbon group may have an appropriate substituent, and various kind groups such as, for example, an alkoxy group, an ester group, an amino group, a heterocyclic group are taken into account.

In addition, as an anion represented by the symbol X in the foregoing formula, it may be various kinds of anions, and can be a pharmacologically acceptable anion. For example, it may be an inorganic acid ion such as a halogen ion or a sulfate ion, or an organic acid ion, or further, a complex ion or the like.

As a fullerene constituting the carbon cluster skeleton in the fullerene derivative of the invention of this application, it may be C60 or a higher order fullerene such as C70 or C82.

In the antimicrobial agent or anticancer agent of the invention of this application, one or more kinds of the fullerene derivatives as above can be contained as the active ingredients.

With regard to the explanation of synthesis of the fullerene derivative as the active ingredient of the invention of this application, as for a fullerene derivative in which R³ is H, and having an alkyl substituent at R⁴ in the foregoing formula, when the case of, for example, n pairs is illustrated, it can be synthesized according to the following reaction formula or the like:

C₆₀ + CH₃NHCH₂CO₂H + RCHO

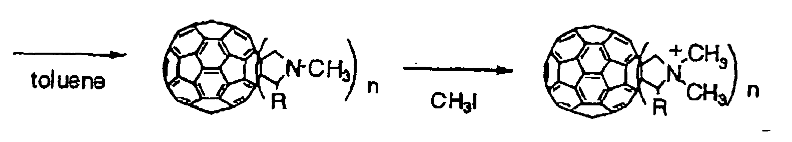

(wherein R denotes an alkyl substituent as the one corresponding to the foregoing R⁴).

In other words, for example, in toluene, C60, N-methylglycine and various aldehydes (RCHO) are dissolved, and heated under argon gas flow, whereby a 2-alkyl-N-methylpyrrolidine derivative is obtained. The reaction time and the amounts of reagents are adjusted according to the number of n of a desired derivative. The product is purified with a silica gel column. Subsequently, in methyl iodide at room temperature or by heating, a derivative having various alkyl substituents (R) (a 2-alkyl-N,N-dimethylpyrrolidinium derivative) can be obtained. Specifically, for example, as described in the following formula, fullerene derivatives, in which R³ is H, and R⁴ is H (derivative 2), C4H9 (derivative 3), C6H13 (derivative 4) and C9H19 (derivative 5) have been synthesized. It has been confirmed that these are dissolved in DMSO at a concentration of 5 mM or more.

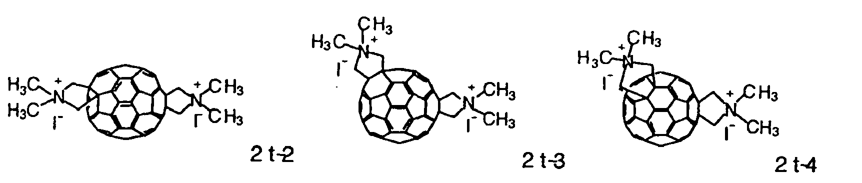

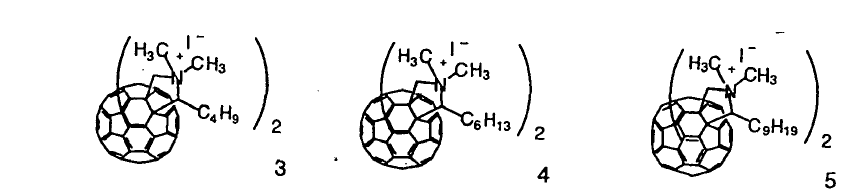

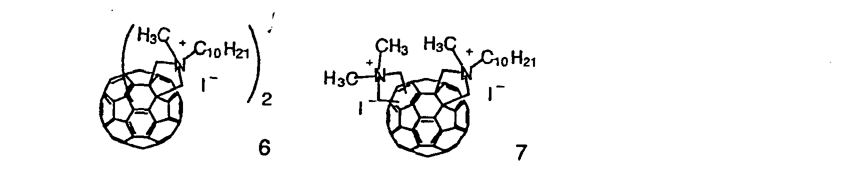

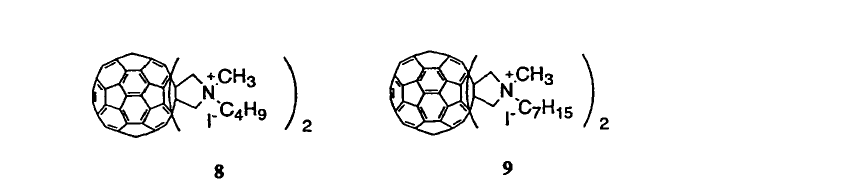

In addition, with regard to a fullerene derivative having an alkyl substituent at R¹ or R², which is bonded to a nitrogen atom in the foregoing formula, in the case of synthesizing it by introducing these alkyl substituents, for example, it can be synthesized according to the following reaction formula or the like.

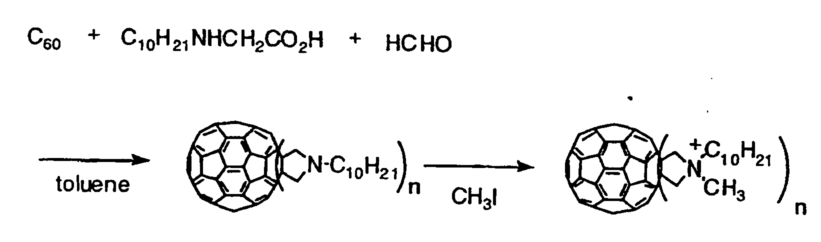

In other words, for example, in toluene, C60, N-decylglycine and formaldehyde are dissolved, and heated under argon gas flow, whereby an N-decylpyrrolidine derivative is obtained. The reaction time and the amounts of reagents are adjusted according to the number of n of a desired derivative. The product is purified with a silica gel column. Subsequently, in methyl iodide at room temperature or by heating, a (N-decyl-N-methylpyrrolidinium derivative) can be obtained.
In this way, specifically, for example, a fullerene derivative of the foregoing derivative 6 is synthesized.

In addition, in the case of synthesizing the foregoing derivative 8, by using "N-butylglycine" instead of "N-decylglycine", further, in the case of synthesizing the foregoing derivative 9, by using "N-heptylglycine" instead of "N-decylglycine", these fullerene derivatives can be synthesized.

With regard to the antimicrobial agent and anticancer agent of the invention of this application, the form is not particularly limited, and for example, it may be a solid preparation such as a tablet, a granule, a fine granule, a pill, a powder, a capsule, a troche or a chewable tablet, a jelly preparation or an adhesive preparation for external use, a liquid preparation such as an elixir, a syrup, a suspension, an emulsion, an injection or a transfusion.

For preparing a preparation, a common carrier component can be used according to the type of preparation. For example, for preparing a solid preparation, a common component, for example, an excipient such as a saccharide including starch, lactose, sucrose, mannitol, cornstarch and the like, crystal cellulose, carboxymethyl cellulose or light silicic acid anhydride; a binder such as polyvinyl alcohol, polyvinyl pyrrolidone, polyvinyl ether, ethyl cellulose, gum arabic, tragacanth, gelatin, hydroxypropyl cellulose, hydroxypropyl methylcellulose, calcium citrate, dextrin or pectin; a lubricant such as magnesium stearate, calcium stearate, talk, polyethylene glycol or colloid silica; a disintegrator such as starch, carboxymethyl cellulose, calcium carboxymethyl cellulose or croscarmellose sodium, a disintegrating aid, a moisturizing agent, a surfactant or the like can be used.

For preparing a liquid preparation, a common component, for example, a solvent such as water for injection, water, ethanol or ethylene glycol, a solubilizing aid such as ethanol, polyethylene glycol, propylene glycol, D-mannitol, cholesterol, triethanolamine, sodium carbonate or sodium citrate, a surfactant such as stearyl triethanolamine, sodium lauryl sulfate, lecithin or glycerin monostearate, a suspending agent for a hydrophilic polymer or the like such as polyvinyl alcohol, polyvinyl pyrrolidone, sodium carboxymethyl cellulose, methyl cellulose, hydroxymethyl cellulose, hydroxyethyl cellulose or hydroxypropyl cellulose, a tonicity adjusting agent such as sodium chloride, glycerin or D-mannitol, a buffering agent such as a phosphate, an acetate, a carbonate or a citrate, a soothing agent such as benzyl alcohol, glucose, an amino acid or the like can be used. For the foregoing solid preparation or liquid preparation, a preservative, a solubilizer, an emulsifier, a dispersant, a thickening agent, a plasticizer, an adsorbent, a flavor, a coloring agent, a corrective, a sweetner, an antiseptic, an antioxidant or the like can be used according to need. The antimicrobial agent of the present invention can be produced, for example, by a common method such as mixing, kneading, granulating, tableting, coating, sterilizing, emulsifying according to the dosage form of preparation. With regard to production of preparations, each provision of General Rules for Preparations in Japanese Pharmacopoeia can be referred to.

In addition, the antimicrobial agent and anticancer agent of the invention of this application may contain other pharmaceutically active substances as long as they contain the foregoing fullerene derivative as the active ingredient.

For example, in the case where it is prescribed as an oral medicine, an antiacid such as sodium hydrogen carbonate, calcium carbonate, magnesium carbonate, magnesium oxide, aluminum hydroxide, aluminum hydroxide gel, magnesium silicate, magnesium aluminosilicate, synthetic aluminum silicate, a coprecipitated product of aluminum hydroxide and sodium hydrogen carbonate, a coprecipitated product of magnesium hydroxide and magnesium carbonate, magnesium aluminometasilicate or dihydroxy aluminum aminoacetate, a stomachic such as gentian, swertia herb, nux vomica, japanese gentian, bitter orange peel, fennel, carnitine chloride, glutamic acid hydrochloride, betaine hydrochloride or bethanechol chloride, a digestive such as pancreatin, pepsin, lemonase chloride, cholic acid, bile powder or dehydrocholic acid, a drug for controlling intestinal function such as mallotus bark, gambir, ubai, cassia seed or geranium herb, an antidiarrheal drug such as loperamide, bismuth subnitrate, bismuth subcarbonate or berberine chloride, an analgesic and antispasmodic such as dicyclomine hydrochloride, scopolamine hydrobromide, atropine methylbromide, scopolamine methylbromide, papaverine hydrochloride, isopropamide iodide, belladonna extract or scopolia extract, a mucosa repairing agent such as sucralfate, sulpiride, gefarnate or teprenone or the like is included. Among these active substances, one or more can be used.

In addition, with regard to the antimicrobial agent of the invention of this application, according to need, a bismuth preparation, metronidazole, tinidazole, or further various antibiotics such as clarithromycin may be combined, and with regard to the anticancer agent of the invention of this application, a hormone therapy agent, a chemotherapy agent, for example, an alkylating agent, an antimetabolite or an anticancer antibiotic, an immunotherapy agent or the like may be contained.

With regard to the antimicrobial agent of the invention of this application, its dose is not particularly limited, however, as a guide, the range between 0.01 and 500 mg per day for an adult can be taken into account, and with regard to the anticancer agent, in the case of oral administration, the range between 0.005 and 50 mg per kilogram of body weight per day in divided doses can be taken into account.

The fullerene derivative of the invention of this application has low toxicity and is useful as a drug.

Of course, in the invention of this application, human application is taken into account, however, it goes without saying that it may apply to non-human animals. The synthesis examples as an example are shown as follows.

### < Synthesis Examples >

### 1: Synthesis of C60-bis(N-methyl-2-hexylpyyrolidine)

In 600 ml of absolute toluene, 200 mg of C60 (0.28 mmol) was dissolved, and 50 mg of N-methylglycine (0.56 mmol, 2 equivalent amounts) and 95 mg of heptanal (0.83 mmol, 3 equivalent amounts) were added, then heated to reflux at 135°C for 3 hours under argon gas flow. The reaction solution was washed with water and saturated saline, dried with sodium sulfate, then the solvent was evaporated under reduced pressure. The obtained solid was purified by silica gel column chromatography (as the eluent, toluene : hexane = 5:1, 100% toluene, toluene : ethyl acetate = 20:5 were used in this order), thus 25 mg of a regioisomer mixture of C60-bis(N-methyl-2-hexylpyyrolidine) (0.025 mmol) was obtained (the yield was 9%).

### 2: Synthesis of C60-bis(N,N-dimethyl-2-hexylpyyrolidinium iodido)

In 5 ml of methyl iodide, 65 mg of C60-bis(N-methyl-2-hexylpyyrolidine) (0.065 mmol) was reacted at room temperature by stirring for 24 hours. The precipitated solid was sequentially washed with toluene and ethyl acetate, thus 55 mg of C60-bis(N,N-dimethyl-2- hexylpyyrolidinium iodido) (0.043 mmol) was obtained. The yield was 65%.

For example, with regard to the antimicrobial agent containing a fullerene derivative as an active ingredient of the invention of this application as mentioned above, first, the antimicrobial activity of this fullerene derivative can be explained with reference to Table 1.

Incidentally, with regard to the foregoing derivative 2, major regioisomers were separated and purified, however, with regard to others, bioactivities were investigated using mixtures.

Table 1 shows the minimum concentration (MIC) of each fullerene derivative which inhibits the growth of various gram positive bacteria in comparison with that of vancomycin (VCM) (the lower the concentration is (small numerical value) the higher the activity is). Each fullerene derivative was dissolved in DMSO, then added to the culture medium of bacteria. Vancomycin is an antimicrobial agent used for drug resistant bacteria (Methicillin resistant strains, MRSA) which has been in trouble now. The regioisomers of the fullerene derivative 2, 2t-2, 2t-3 and 2t-4, and 3, 4, 7 and 8 showed an effective antimicrobial activity, however, the activities of 3, 4 and 8 were somewhat lower than those of the regioisomers of the fullerene derivative 2 and the fullerene derivative 7. Among the regioisomers of the fullerene derivative 2, 2t-2, 2t-3 and 2t-4, a difference in the effects is observed in some degree depending on bacteria, however, there are no significant differences, and they have an antimicrobial activity substantially equivalent to that of vancomycin, and are also effective for MRSA. In particular, it is notable that an extremely high antimicrobial activity is observed in these fullerene derivatives. Furthermore, worthy of special mention is the fact that these fullerene derivatives show an effective antimicrobial activity against also vancomycin-resistant bacteria (VRE). In addition, the fact that there are no differences among the regioisomers of the fullerene derivative 2 indicates that the regioisomers of the other derivatives do not need to be separated. At present, vancomycin-resistant bacteria began to emerge, therefore, the fullerene derivative 2, the fullerene derivative 7 or the like is expected to exert an effect on these.

**Table 1 MIC (mg/mL) of fullerene for gram positive bacteria**

| | 2t-2 | 2t-3 | 2t-4 | 3 | 4 | 7 | 8 | VCM |
|---|---|---|---|---|---|---|---|---|
| S. aureus 209P JC-1 | 1.56 | 0.78 | 3.12 | 6.25 | 6.25 | 0.39 | 6.25 | 1.56 |
| S. aureus M133 (MRSA) | 0.78 | 1.56 | 3.12 | 6.25 | 12.5 | 0.39 | 6.25 | 1.56 |
| S. aureus M126 (MRSA) | 3.12 | 1.56 | 3.12 | 6.25 | 12.5 | 0.78 | 6.25 | 1.56 |
| S. eidermidis ATCC 14990 | 6.25 | 3.12 | 3.12 | 6.25 | 12.5 | 0.39 | 3.12 | 3.12 |
| E. hirae ATCC 8043 | 12.5 | 6.25 | 6.25 | 6.25 | 25 | 1.56 | 6.25 | 3.12 |
| E. faecalis W-73 | 12.5 | 6.25 | 6.25 | 6.25 | 50 | 0.39 | 6.25 | 3.12 |
| E. faecium vanA (VRE) | 12.5 | 6.25 | 6.25 | 12.5 | 12.5 | 0.39 | 6.25 | >100 |
| E. faecalis NCTC 12201 (VRE) | 12.5 | 3.12 | 6.25 | 6.25 | 25 | 0.39 | 6.25 | >100 |

On the other hand, in the invention of this application, it is also emphasized that the foregoing fullerene derivatives have an anticancer activity against a wide variety of cancer cells, for example, cancer cells of breast cancer, brain tumor, colon cancer, lung cancer, stomach cancer, ovarian cancer, kidney cancer, prostate cancer and the like.

With regard to this anticancer activity, the inventor found the effect of a fullerene derivative on growth of cancer cells in the panel experiment of cultured human cancer cells. In this experiment, concentrations of the compounds inhibiting growth of 37 kinds of cultured human cancer cells by 50 % (GI 50) are measured and the mean value (MID-GI 50) is obtained, and pattern analysis is performed for differences between MID-GI 50 and GI 50 value for each cancer cell. Generally, MID-GI 50 shows the potency of the compound as an anticancer agent. In addition, in the case where the result of the foregoing pattern analysis is similar to that of an anticancer agent that is currently used, it can be pointed out that the action mechanism is similar to that of an existing anticancer agent. On the contrary, in the case where the pattern is different from that of an existing anticancer agent, there is a possibility that it may be an anticancer agent which is based on a novel mechanism.

In a panel screening, it is evaluated that in the case where MID-GI 50 is -5 or less, it is promising, in the case where the highest numerical value of correlation coefficient r, r max, is 0.5 or less, it is a novel action mechanism, and in the case of 0.5 < r max < 0.75, a possibility of a novel action mechanism is high. The regioisomers of the fullerene derivative 2 of the invention of this application and 3, 4 and 5 are effective from the results of MID-GI 50 of -5 or less, and also there was a possibility that their action mechanisms are new. In addition, with regard to 6 and 7, though their activities are somewhat low, their action mechanisms are considered to be entirely new. As mentioned above, there is a possibility that any derivative is a promising anticancer agent.

The evaluation results are shown in the following Table 2.

**Table 2**

| Fullerene | MID-GI 50 | Highest correlation coefficient (rₘₐₓ) |
|---|---|---|
| 2t-2 | -5.44 | 0.490 |
| 2t-3 | -5.18 | 0.523 |
| 2t-4 | -5.18 | 0.534 |
| 3 | -5.42 | 0.701 |
| 4 | -5.48 | 0.709 |
| 5 | -5.61 | 0.637 |
| 6 | -4.79 | 0.493 |
| 7 | -4.74 | 0.438 |

### Industrial Appliciably

As described in detail above, by the invention of this application, a novel antimicrobial agent and anticancer agent comprising, as an active ingredient, a fullerene derivative having a high antimicrobial activity and anticancer activity is provided.

## Claims

1. An antimicrobial agent or anticancer agent, **characterized by** comprising, as an active ingredient, a fullerene derivative which has an organic bond structure represented by the following formula: 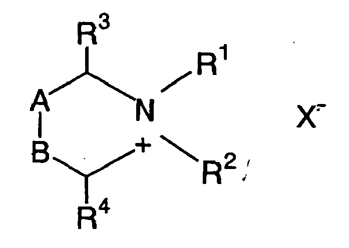
(wherein A and B denote adjacently bonded carbon atoms, R¹ and R² are identical or different and denote a hydrocarbon group which may have a substituent, R³ and R⁴ are identical or different and denote a hydrogen atom or a hydrocarbon group which may have a substituent, and X denotes an anion) attached to respective plural pairs of adjacent bonded carbon atom pairs constituting the carbon cluster skeleton of the fullerene.

2. The antimicrobial agent or anticancer agent according to claim 1 or 2, **characterized in that** the hydrocarbon group is a linear chain, branched chain or cyclic hydrocarbon group.

3. The antimicrobial agent or anticancer agent according to claim 2, **characterized in that** the hydrocarbon group is an alkyl group having 1 to 16 carbon atoms.

4. The antimicrobial agent or anticancer agent according to any one of claims 1 to 3, **characterized in that** the anion is at least one kind of inorganic acid ions and organic acid ions.

5. The antimicrobial agent or anticancer agent according to any one of claims 1 to 4, **characterized in that** the fullerene is at least one kind of C60 and higher order fullerenes.
